# EUROPEAN PATENT APPLICATION

(11) **EP 4 847 919 A1**
(43) Date of publication of application: **02.12.2026**
(21) Application number: 25206210.4
(22) Date of filing: 02.10.2025
(51) Int. Cl.: G01N 33/542

(54) **RECOMBINANT STAT PROTEIN AND METHOD TO INTERROGATE STAT INTERACTION AND ACTIVATION**

(30) Priority: 26.05.2025 EP 25178814
(71) Applicant: Universität Zürich, 8006 Zürich (CH)
(72) Inventor: Boyman, Onur, 8044 Zürich (CH); Meledin, Roman, 8044 Zürich (CH); Nguyen Anh Nguyen, Thi, 8044 Zürich (CH)
(74) Representative: Schulz Junghans Patentanwälte PartGmbB

(57) **Abstract**

The invention relates to a method for observing signal transducer and activator of transcription (STAT) dimerization by:
a. Providing a cell that recombinantly expresses a first and a second variant of a STAT polypeptide, each covalently linked to a peptide capable of serving as either donor or acceptor in a FRET pair. The first variant is attached to a first fluorescent peptide moiety linked to the STAT polypeptide, and the second variant is attached to a second fluorescent peptide moiety. At least one of the first variant and the second variant consist of a truncated STAT polypeptide. The first and the second peptide moiety linked to the first and second STAT variants, respectively, form a Förster resonance energy transfer (FRET) pair comprising a resonance energy donor and a resonance energy acceptor.
b. Measuring Förster resonance energy transfer (FRET) between the first fluorescent moiety and the second fluorescent moiety.

A second aspect of the invention relates to expression systems and constructs employed in the method according to the invention.

## Description

### Field

The present invention relates to fluorescently modified STAT polypeptides, polynucleotides encoding same, and to methods for using the polypeptides and polynucleotides of the invention in the discovery and characterization of pharmaceutical compounds targeting STAT signal transduction.

### Background

The signal transducer and activator of transcription (STAT) family comprises seven members in humans, including STAT1, STAT2, STAT3, STAT4, STAT5A, STAT5B, and STAT6. STAT proteins are structurally composed of six domains: the N-terminal domain (NTD), coiled-coil domain (CCD), DNA-binding domain (DBD), linker domain (LD), src homology-2 (SH2) domain, and C-terminal transactivation domain (TAD). The CCD, DBD, LD and SH2 domains are collectively referred to as the core fragment (CF) of STATs.

STAT5 is a central member of a signalling cascade that can be triggered by several extracellular stimuli, including cytokines, growth factors, and hormones. Following stimulation with their cognate ligands, the intracellular domains of cell surface receptors undergo tyrosine phosphorylation by Janus kinases (JAKs). Phosphorylated receptor intracellular domains then serve as a docking platform for inactive unphosphorylated STAT5 (uSTAT5), which at steady state exists as monomer or 'anti-parallel' dimer. Upon binding the intracellular domain of the activated cell surface receptor, uSTAT5 becomes phosphorylated and, as observed in other STATs, presumably undergoes a conformational change to form active 'parallel' dimers or tetramers. Subsequently, phosphorylated STAT5 (pSTAT5) dimers translocate to the nucleus where they initiate transcription of target genes. Inactivation of pSTAT5 occurs either by its cognate phosphatases or via the ubiquitin-proteosome degradation machinery.

Delicate control of STAT5 activation is crucial for the proliferation, metabolism, differentiation and survival of immune, epithelial and mesenchymal cells. Consequently, imbalanced STAT5 activity can cause numerous human diseases, including cancer, autoimmunity, and immunodeficiency, making STAT5 and associated upstream signalling proteins valuable drug targets. Thus, several JAK inhibitors have been approved for the treatment of various immune-mediated diseases and malignancies. However, the development of drugs targeting STATs and STAT5 in particular is less advanced, partly due to a lack of appropriate tools for sensing STAT activation in cellular context.

Measuring STAT5 activation is useful to investigate the functionality of immune cells and the efficacy of newly developed drug compounds. Typically, the activity of the canonical JAK-STAT5 pathway is measured by detecting phospho-tyrosine (pTyr) residues 694 and 699 of STAT5A and STAT5B, respectively, by intracellular staining using pSTAT5-specific antibodies. However, since this method requires fixation and permeabilization of the cells assessed, it does not allow continuous real-time monitoring of STAT5 activation in live cells. A similar limitation applies to commercially available reporter cell lines, which typically contain a reporter gene under the control of a promoter fused to the STAT5 binding site. Biosensors serve as powerful tools to monitor protein signaling cascades in living systems.

Examples of these biosensors include STAT reporter assays based on bimolecular fluorescence complementation (BiFC; Letra-Vilela et al. FASEB Bioadv 2, 116-125 (2020)) or the homoFluoppi (Okada et al. Sci Rep 8, 2385 (2018)), which were used to detect STAT3 dimerization. However, BiFC suffers from low time resolution and signal-to-noise ratios, and homoFluoppi is unsuitable for monitoring STAT heterodimerization. Förster resonance energy transfer (FRET) is a useful approach to assess oligomerization and conformational change of proteins continuously and reversibly with a high spatiotemporal resolution. In a proof-of-principle experiment, STAT5 dimerization has been confirmed by ratiometric FRET in a non-continuous manner (Neculai et al. Journal of Biological Chemistry 280, 40782-40787 (2005)). To date, there is no approach available for continuous real-time monitoring of STAT5 activation in living systems.

Based on the above-mentioned state of the art, the objective of the present invention is to provide means and methods to interrogate STAT protein interaction in-vitro. This objective is attained by the subject-matter of the independent claims of the present specification, with further advantageous embodiments described in the dependent claims, examples, figures and general description of this specification.

### Summary of the Invention

The inventors developed a class of highly sensitive genetically-encoded STAT biosensors, which allowed direct and continuous detection of STAT activity in live cells with high spatiotemporal resolution. Using human STAT5A, they demonstrate the versatility of their system to quantify the activation of wild-type STAT5 versus disease-associated STAT5 mutations and to precisely select compounds targeting the STAT5 signalling pathway. Moreover, one example of the biosensors disclosed herein also facilitated real-time tracking of STAT5 activation in human primary CD4+ T cells. Collectively, these novel biosensors open up new possibilities of studying STAT biology and druggability in various cellular contexts.

A first aspect of the invention relates to a method for observing STAT dimerization in a cell ex-vivo by the following steps:
a. Providing a cell that recombinantly expresses a first variant and a second variant of a STAT polypeptide, each being covalently linked to a peptide capable of serving as either a donor or acceptor in a FRET pair. The first variant is attached to a first (directly or indirectly fluorescent) peptide moiety covalently linked to the STAT polypeptide, and the second variant is attached to a second (directly or indirectly fluorescent) peptide moiety covalently linked to the STAT polypeptide. At least one of the first variant and the second variant consists of a truncated STAT.
   The first peptide moiety and the second peptide moiety linked to the first and second STAT variants, respectively, form a Förster resonance energy transfer (FRET) pair comprising a resonance energy donor and a resonance energy acceptor.
b. Measuring Förster resonance energy transfer (FRET) between the first fluorescent moiety and the second fluorescent moiety.

A second aspect of the invention relates to expression systems and constructs employed in the method according to the invention.

### Terms and definitions

### General

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term FRET pair in the context of the present specification relates to a pair of fluorescent (or, in the case of BRET, luminescent) proteins or -moieties capable of Förster Resonance Energy Transfer (FRET), where energy is transferred non-radiatively from an excited donor fluorescent molecule to an acceptor fluorescent molecule through dipole-dipole coupling, typically over distances of 1-10 nm. In the context of fluorescent proteins, FRET occurs when the emission spectrum of a donor protein overlaps with the absorption spectrum of an acceptor protein, enabling the transfer of energy between this FRET pair.

Any patent document cited herein shall be deemed incorporated by reference herein in its entirety.

### Sequences

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

In the context of the present specification, the terms *sequence identity* and *percentage of sequence identity* refer to a single quantitative parameter representing the result of a sequence comparison determined by comparing two aligned sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless stated otherwise, sequence identity values provided herein refer to the value obtained using the BLAST suite of programs (Altschul et al., J. Mol. Biol. 215:403-410 (1990)) using the above identified default parameters for protein and nucleic acid comparison, respectively.

Reference to identical sequences without specification of a percentage value implies 100% identical sequences (i.e. the same sequence).

Particular embodiments make use of the sequences as disclosed herein (i.e. 100% identical).

### General Biochemistry: Peptides, Amino Acid Sequences

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

The term *peptide* or oligopeptide in the context of the present specification relates to a molecule consisting of up to 50 amino acids, in particular 8 to 30 amino acids, more particularly 8 to 15 amino acids, that form a linear chain wherein the amino acids are connected by peptide bonds.

Amino acid residue sequences are given from amino to carboxyl terminus. Capital letters for sequence positions refer to L-amino acids in the one-letter code (Stryer, Biochemistry, 3^{rd} ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The term *variant* refers to a polypeptide that differs from a reference polypeptide, but retains essential properties. A typical variant of a polypeptide differs in its primary amino acid sequence from another, reference polypeptide. Generally, differences are limited so that the sequences of the reference polypeptide and the variant are closely similar overall and, in many regions, identical. A variant and reference polypeptide may differ in amino acid sequence by one or more modifications (e.g., substitutions, additions, and/or deletions). A substituted or inserted amino acid residue may or may not be one encoded by the genetic code. A variant of a polypeptide may be naturally occurring such as an allelic variant, or it may be a variant that is not known to occur naturally.

The term variant as used herein refers to STAT variants that are identical to the parent STAT molecule but truncated, i.e. have a part of their protein sequence deleted. The variants disclosed herein are linked covalently to a fluorescent protein moiety.

In the context of the present specification, the term *amino acid linker* or *peptide linker* refers to a polypeptide of variable length that is used to connect two polypeptides in order to generate a single chain polypeptide. Exemplary embodiments of linkers useful for practicing the invention specified herein are oligopeptide chains consisting of 1, 2, 3, 4, 5, 10, 20, 30, 40 or 50 amino acids, particularly 1, 2, 3, 4, 5 amino acids.

There is no constraint on the amino acid composition of the linker. In certain embodiments, the linker consists of amino acids selected from the group of G S, A and D. An important characteristic of the conjugate peptide linkers as specified above are low immunogenicity, and a peptide length that allows the domains which are joined by the linker, to interact to form a functional entity as disclosed herein. In particular desirable embodiments of the domain peptide linkers specified above, the sequences are primarily made up of stretches of amino acids such as glycine (G) and serine (S).

### General Molecular Biology: Nucleic Acid Sequences, Expression

The term *transgene* in the context of the present specification relates to a gene or genetic material that has been transferred from one organism to another. In the present context, the term may also refer to transfer of the natural or physiologically intact variant of a genetic sequence into tissue of a patient where it is missing. It may further refer to transfer of a natural encoded sequence the expression of which is driven by a promoter absent or silenced in the targeted tissue.

The term *recombinant* in the context of the present specification relates to a nucleic acid, which is the product of one or several steps of cloning, restriction and/or ligation and which is different from the naturally occurring nucleic acid. A recombinant virus particle comprises a recombinant nucleic acid. Expressing a gene or protein "recombinantly" refers to recombinant expression in the sense of the preceding paragraph.

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of a RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

The term "expression cassette" refers to a nucleic acid construct comprising a coding sequence operably linked to a promoter and a termination sequence, wherein the promoter drives transcription of the coding sequence and the termination sequence ensures proper termination and, in the case of mRNA expression, polyadenylation of the transcript in a eukaryotic host cell.

The term *nucleic acid expression vector* in the context of the present specification relates to a plasmid, a viral genome or an RNA, which is used to transfect (in case of a plasmid or an RNA) or transduce (in case of a viral genome) a target cell with a certain gene of interest, or -in the case of an RNA construct being transfected- to translate the corresponding protein of interest from a transfected mRNA. For vectors operating on the level of transcription and subsequent translation, the gene of interest is under control of a promoter sequence and the promoter sequence is operational inside the target cell, thus, the gene of interest is transcribed either constitutively or in response to a stimulus or dependent on the cell's status. In certain embodiments, the viral genome is packaged into a capsid to become a viral vector, which is able to transduce the target cell.

The term *fluorescent protein* in the context of the present specification may relate, but is not limited to, a fluorescent protein selected from the group comprising:
- green fluorescent protein (GFP) from *Aequorea victoria* and derivatives thereof, such as
   - enhanced blue fluorescent protein (EBFP), enhanced blue fluorescent protein 2 (EBFP2), azurite, mKalama1, Sirius;
   - enhanced green fluorescent protein (EGFP), emerald, superfolder avGFP, T-sapphire;
   - yellow fluorescent protein (YFP), enhanced yellow fluorescent protein (EYFP), citrine, venus, YPet, topaz, SYFP, mAmetrine;
   - enhanced cyan fluorescent protein (ECFP), mTurquoise, mTurquoise2, cerulean, CyPet, SCFP;
- fluorescent protein from *Discosoma striata* and derivatives thereof:
   - mTagBFP;
   - TagCFP, AmCyan, Midoriishi Cyan, mTFP1;
   - Azami Green, mWasabi, ZsGreen, TagGFP, TagGFP2, TurboGFP, CopCFP, AceGFP;
   - TagYFP, TurboYFP, ZsYellow, PhiYfP;
   - Kusabira Orange, Kusabira Orange2, mOrange, mOrange2, dTomato, dTomato-Tandem, DsRed, DsRed2, DsRed-Express (T1), DsRed-Express2, DsRed-Max, DsRed-Monomer, TurboRFP, TagRFP, TagRFP-T;
   - mRuby, mApple, mStrawberry, AsRed2, mRFP1, JRed, mCherry, eqFP611, tdRFP611, HcRed1, mRaspberry;
   - tdRFP639, mKate, mKate2, katushka, tdKatushka, HcRed-Tandem, mPlum, AQ143.
- proteins derived from alpha-allophycocyanin from the cyanobacterium *Trichodesmium erythraeum* such as small ultra-red fluorescent protein (smURFP).
- The fluorescent protein moieties disclosed in the Example section are similarly encompassed by the term fluorescent protein.

### Detailed Description of the Invention

A first aspect of the invention relates to a method for observing STAT dimerization in a cell ex-vivo.

This method comprises the following steps:
a. Providing a cell that recombinantly expresses a first variant of a STAT polypeptide and a second variant of a STAT polypeptide, each being covalently linked to a peptide moiety that directly or indirectly is capable of serving as either a donor or acceptor in a FRET pair. The first variant is attached to a first fluorescent peptide moiety covalently linked to the STAT polypeptide, and the second variant is attached to a second fluorescent peptide moiety covalently linked to another polypeptide chain of the same type of STAT polypeptide. At least one of the first variant and the second variant consists of a truncated STAT polypeptide.
   The first fluorescent peptide moiety and the second fluorescent peptide moiety linked to the first and second STAT variants, respectively, form a Förster resonance energy transfer (FRET) pair comprising a resonance energy donor and an resonance energy acceptor.
b. Measuring Förster resonance energy transfer (FRET) between the first fluorescent moiety and the second fluorescent moiety.

The two variants are expressed each as a transgene. In some embodiments, the cell does not express the wild-type variant of the STAT polypeptide being made subject of the method.

### Fluorescent moieties:

As a principle, the first and the second fluorescent moiety are different, selected to form a Förster resonance energy transfer (FRET) pair. In certain embodiments, the first and the second fluorescent moiety is each a fluorescent protein, selected to absorb and emit at wavelengths suitable for FRET. Several donor-acceptor fluorescent protein pairs suitable for FRET are given herein by way of example, and more are known to the skilled person.

In addition to the combinations used in the Example section, commonly used FRET pairs with commercially available fluorescent proteins include, but are not limited to:
- *1. CFP (Cyan Fluorescent Protein) and YFP (Yellow Fluorescent Protein)* Donor: CFP (Cyan, ~480 nm excitation, ~510 nm emission) Acceptor: YFP (Yellow, ~515 nm excitation, ~530 nm emission)
- *2. GFP (Green Fluorescent Protein) and mCherry* Donor: GFP (Green, -488 nm excitation, ~509 nm emission) Acceptor: mCherry (Red, ~587 nm excitation, ~610 nm emission)
- *3. mTurquoise2 and mApple* Donor: mTurquoise2 (Blue, ~434 nm excitation, ~475 nm emission) Acceptor: mApple (Red, ~573 nm excitation, ~595 nm emission)
- *4. mVenus and mRuby2* Donor: mVenus (Yellow, ~515 nm excitation, ~528 nm emission) Acceptor: mRuby2 (Red, ~577 nm excitation, ~605 nm emission)
- *5. mCerulean3 and mCitrine* Donor: mCerulean3 (Blue, ~433 nm excitation, ~475 nm emission) Acceptor: mCitrine (Yellow, ~514 nm excitation, ~529 nm emission)
- *6. mTagBFP and mApple* Donor: mTagBFP (Blue, ~400 nm excitation, ~454 nm emission) Acceptor: mApple (Red, ~573 nm excitation, ~595 nm emission)
- *7. mOrange and mStrawberry* Donor: mOrange (Orange, ~548 nm excitation, ~560 nm emission) Acceptor: mStrawberry (Red, ~574 nm excitation, ~605 nm emission)

These pairs are widely available as recombinant proteins from various commercial sources such as Clontech (Takara Bio), Invitrogen (Thermo Fisher), and Addgene.

Likewise, the donor may be selected from the pool of bioluminescent proteins operating at suitable wavelengths. Bioluminescence resonance energy transfer (BRET) pairs suitable for studying protein-protein interactions include combinations such as Renilla luciferase (RLuc) with yellow fluorescent protein (YFP), NanoLuc^{®} luciferase with NanoBRET^{®} 618 fluorophore, and RLuc with enhanced GFP variants like EYFP. These pairs are optimized based on spectral overlap between the donor's bioluminescent emission and the acceptor's excitation spectrum, ensuring efficient resonance energy transfer within proximity (<10 nm). NanoLuc^{®}, being smaller and brighter than other luciferases, reduces steric hindrance and enhances signal-to-noise ratio, especially when paired with red-shifted fluorophores. Such pairs are ideal for real-time, live-cell assays of protein interactions due to their high sensitivity and applicability in complex cellular environments.

A further possibility is to use small so-called chemogenetic tags instead of the larger fluorescent or luminescent proteins. Chemogenetic tags such as CLIP-tag, SnapTag, and HaloTag offer versatile alternatives to fluorescent proteins for studying protein-protein interactions through FRET. These self-labelling tags enable covalent attachment of synthetic fluorophores with optimized photophysical properties, providing greater flexibility in selecting donor-acceptor pairs. For instance, CLIP-tag reacts specifically with benzylcytosine ligands, while HaloTag binds chloralkane derivatives, allowing precise labelling of proteins with compatible fluorophores like rhodamines or red dyes. These tags improve FRET efficiency by enabling site-specific labelling and minimizing non-specific background signals. Their compatibility with live-cell imaging and high-throughput assays makes them ideal for dynamic studies of protein interactions in complex biological environments.

Self-labelling tags are described in detail in Keppler et al., A general method for the covalent labeling of fusion proteins with small molecules in vivo. Nat. Biotechnol. 2003, 21 (1), 86-9; Correa et al., Substrates for improved live-cell fluorescence labelling of SNAP-tag. Current pharmaceutical design 2013, 19 (30), 5414-5420; Wilhelm et al., Kinetic and Structural Characterization of the Self-Labeling Protein Tags HaloTag7, SNAP-tag, and CLIP-tag. Biochemistry 2021, 60 (33), 2560-2575; Gautier et al. An engineered protein tag for multiprotein labelling in living cells. Chem. Biol. 2008, 15 (2), 128-36. Substrates of engineered AGT are disclosed, inter alia, in US8367361B2 to Johnsson et al. (also published as WO02083937A2; the first SNAP tag patent); US8227602B2 to Gautier et al. (also published as WO2008012296A1; CLIP and CLIP substrates); US8163479B2 to Jaccard et al.; US8178314B2 to Kindermann et al., all of which are incorporated by reference in their entirety herein.

An alternative is the FIAsH tag, the FlAsH/tetracysteine (TC) tag, a genetically encoded peptide tag that binds specifically to fluorescein arsenical hairpin (FlAsH) dyes. This technology uses a small tetracysteine motif engineered into target proteins, which reacts with biarsenical dyes like FlAsH or ReAsH to produce fluorescence. Flash tags are advantageous for protein labelling due to their small size, which minimizes disruption to protein function, and their ability to label proteins internally or at termini. They are particularly useful for imaging protein dynamics and interactions in living cells, offering an alternative to larger fluorescent proteins with improved spectral properties and reduced steric hindrance.

An alternative of this first aspect of the invention is a method for determining a modulation of STAT dimerization, said method comprising observing STAT dimerization in a cell according to the method laid out above, wherein the cell is exposed to a stimulus; and then comparing the STAT dimerization to a control wherein the stimulus is absent.

The inventors recorded a baseline prior to exposure to STAT modulators, in the same cells, but it will be equally possible to assay treated cells, and then compare the resultant reading to a measurement in untreated controls. In other words: The measurement does not necessarily require two points measured in the same cell population to render a useful output. It would be possible to compare inhibitor treatment with untreated controls without recording the baseline.

The inventors in the course of their investigations measured FLIM in cells after 30 min of interleukin (IL)-2 stimulation.

In certain embodiments, the stimulus is the presence of a compound putatively capable of modulating STAT signaling.

In certain embodiments, the control is a measurement of the same cell prior to adding the stimulus.

Förster Resonance Energy Transfer (FRET) is a non-radiative energy transfer mechanism between two chromophores-a donor and an acceptor-via dipole-dipole coupling. The donor molecule, in its excited state, transfers energy to the acceptor molecule when they are in close proximity (typically 1-10 nm). This process is highly distance-dependent, with efficiency inversely proportional to the sixth power of the distance. FRET is widely used as a molecular ruler in biological and chemical studies to investigate interactions and conformational changes at the nanoscale.

Bioluminescence Resonance Energy Transfer (BRET) is a variation of FRET that uses a bioluminescent protein (e.g., luciferase) as the energy donor and a fluorescent protein as the acceptor. The donor emits light through a biochemical reaction (e.g., oxidation of luciferase's substrate), which excites the acceptor via resonance energy transfer. This technique is commonly employed for studying protein-protein interactions in live cells and offers advantages such as avoiding photobleaching and autofluorescence issues associated with fluorescence-based methods.

In certain embodiments, FRET is measured by fluorescence life-time imaging microscopy (FLIM).

Alternatives to FLIM for measuring FRET include sensitized emission, acceptor photobleaching, spectral imaging, and photobleaching FRET. Sensitized emission detects acceptor fluorescence after donor excitation and requires crosstalk correction, making it suitable for dynamic live-cell experiments, similar to FLIM. Acceptor photobleaching measures donor fluorescence recovery after irreversible acceptor destruction, providing direct FRET efficiency but limited to single-time measurements. Spectral imaging captures full emission spectra for signal unmixing, improving accuracy compared to sensitized emission FRET but requiring advanced equipment. Photobleaching FRET analyses donor photobleaching decay rates in the presence of an acceptor, offering a simpler, although rather semi-quantitative alternative without lifetime measurements.

In certain embodiments, the first variant and the second variant are both truncated on their C terminus. C-terminally truncated STAT variants delivered the best results in the assays performed by the inventors.

In certain embodiments, the first variant does not comprise the C-terminal domain of the STAT wild-type protein, and the second variant does not comprise the C-terminal domain of the STAT wild-type protein; in other words, both partners for the interaction are constituted by the full STAT protein minus their C-terminal domains.

In particular embodiments, the first variant is attached to the first fluorescent moiety at the C terminus of the first, truncated STAT variant, and the second variant is attached to the second fluorescent moiety at the C terminus of the second, truncated STAT variant.

In certain embodiments, the first variant and the second variant both consist of a C-terminally truncated STAT protein and are both ligated each to a fluorescent protein moiety, the first variant and the second variant being linked to a donor fluorophore and an acceptor fluorophore, respectively, the donor and acceptor fluorophore forming a FRET pair.

In certain embodiments, the first variant and the second variant both consist of the five N-terminal domains of the STAT protein and are both ligated each to a fluorescent protein moiety on their C terminus, the first variant and the second variant being linked to a donor fluorophore and an acceptor fluorophore, respectively, the donor and acceptor fluorophore forming a FRET pair.

In certain embodiments, the fluorescent protein moiety is linked to the C-terminally truncated STAT protein through a peptide linker 1 - 4 amino acids in length.

In certain embodiments, the length of the peptide linker is longer than 4 amino acids.

In certain embodiments, the peptide linker comprises proline. Other proline linker designs include (PX)ₙ, (PXXP)ₙ. A common cysteine-containing linker useful for practice in the invention is (CXXXXC)ₙ.

In certain embodiments, a rigid amino acid linker that minimizes structural flexibility and helps maintain a defined spatial orientation between the STAT and fluorescent protein moieties, a helical (rigid) linker is chosen, for example a rigid linker based on EAAAK repeats, which promote α-helical structure and reduce flexibility. Glutamic acid (E) and lysine (K) form intrahelical salt bridges that stabilize the α-helix. Alanine (A) favours helix formation and contributes to rigidity. This sequence promotes a straight, rod-like structure, ideal for separating functional domains while preventing undesired interactions or folding.

In certain embodiments, no linker is present.

The first variant and the second variant may be expressed from different nucleic acid expression cassettes. These expression cassettes may be present on expression vectors, e.g. expression plasmids, in the cell, or may be integrated into the genome of a cell.

In the Examples of the present specification, the acceptor and donor constructs were expressed in two separate vectors. However, it is also possible to express these constructs in a bicistronic vector (flanked by IRES or P2A/T2A sequences) to enable a homogenous expression ratio. In an alternative embodiment not shown herein, the inventors generated a stable cell line expressing the exemplary biosensor (STATeLight5A), where virtually all cells have the same level of biosensor expression and ratio of acceptor to donor.

### Expression level of the two variants

In certain embodiments, the first variant and the second variant with their respective attached fluorescent peptide moieties are expressed from different nucleic acid expression vectors.

In certain embodiments, an expression level of the first variant being attached to a donor fluorophore is between 1% and 95% of an expression level of the second variant being attached to an acceptor fluorophore.

In general, the expression of acceptor should be equal or higher than the expression of donor. The expression level of donor can be much lower than 50% of the expression level of acceptor, as long as it can be technically detected by the confocal microscope. In certain embodiments, the expression level of the first variant comprising a donor fluorophore is between 10% and 90% of an expression level of the second variant comprising an acceptor fluorophore.

### STAT Protein variants

In certain embodiments, the first variant of a STAT polypeptide and the second variant of a STAT polypeptide are variants of STAT5A.

In certain embodiments, the first variant of a STAT polypeptide and the second variant of a STAT polypeptide are variants of STAT5B.

In certain embodiments, the first variant of a STAT polypeptide and the second variant of a STAT polypeptide are variants of STAT1.

In certain embodiments, the first variant of a STAT polypeptide and the second variant of a STAT polypeptide are variants of STAT2.

In certain embodiments, the first variant of a STAT polypeptide and the second variant of a STAT polypeptide are variants of STAT3.

In certain embodiments, the first variant of a STAT polypeptide and the second variant of a STAT polypeptide are variants of STAT4.

In certain embodiments, the first variant of a STAT polypeptide and the second variant of a STAT polypeptide are variants of STAT6.

### Systems and constructs for practicing the method of the invention

In general, any system in which the STAT interaction can be observed, lends itself to practicing the invention. Eukaryotic cells may be expected to most faithfully reproduce the STAT expression and transport phenomena relevant to STAT and STAT modulators in mammalian systems.

In certain embodiments, the cell is a eukaryotic cell.

Also encompassed by the invention is an expression cassette comprising a nucleic acid sequence encoding a C-truncated STAT protein ligated to a fluorescent protein moiety through an optional peptide linker under control of a promoter sequence operable in a mammalian cell.

Another aspect of the invention relates to an expression system comprising:
a. a first expression cassette comprising a nucleic acid sequence encoding a first variant of a STAT polypeptide covalently linked to a first fluorescent peptide moiety and
b. a second expression cassette comprising a nucleic acid sequence encoding a second variant of the same STAT polypeptide covalently linked to a second fluorescent peptide moiety,
   wherein at least one of the first variant and the second variant consist of a truncated STAT polypeptide, and
   wherein the first fluorescent moiety and the second fluorescent peptide moiety can form a Förster resonance energy transfer (FRET) pair comprising a donor fluorophore protein moiety and an acceptor fluorophore protein moiety;
   wherein both the first and second expression cassette are under control of a promoter sequence operable in a mammalian cell.

In certain embodiments, the first variant and/or second variant are linked to the fluorescent peptide protein moiety through a peptide linker.

In certain embodiments, the expression cassette expressing the acceptor fluorophore expresses at higher expression rate than the expression cassette expressing the donor fluorophore.

In certain embodiments, the first variant and the second variant are both truncated on their C terminus.

In certain embodiments, the first variant does not comprise the C-terminal domain of the STAT wild-type protein, and the second variant does not comprise the C-terminal domain of the STAT wild-type protein.

In certain embodiments, the first variant is attached to the first fluorescent moiety at the C terminus of the truncated STAT first variant, and the second variant is attached to the first fluorescent moiety at the C terminus of the truncated STAT second variant.

In certain embodiments, the first variant and the second variant both consist of a C-terminally truncated STAT protein and are both ligated each to a fluorescent protein moiety, the first variant and the second variant being linked to a donor fluorophore and an acceptor fluorophore, respectively, the donor and acceptor fluorophore forming a FRET pair.

In certain embodiments, the fluorescent protein moiety is linked to the C-terminally truncated STAT protein through a peptide linker 1 - 4 amino acids in length.

In certain embodiments, the first variant of a STAT polypeptide and the second variant of a STAT polypeptide are variants of STAT5A, particularly of human origin.

In certain embodiments, the first variant of a STAT polypeptide and the second variant of a STAT polypeptide are variants of STAT5B, particularly of human origin.

In certain embodiments, the first variant of a STAT polypeptide and the second variant of a STAT polypeptide are variants of STAT1, particularly of human origin.

In certain embodiments, the first variant of a STAT polypeptide and the second variant of a STAT polypeptide are variants of STAT2, particularly of human origin.

In certain embodiments, the first variant of a STAT polypeptide and the second variant of a STAT polypeptide are variants of STAT3, particularly of human origin.

In certain embodiments, the first variant of a STAT polypeptide and the second variant of a STAT polypeptide are variants of STAT4, particularly of human origin.

In certain embodiments, the first variant of a STAT polypeptide and the second variant of a STAT polypeptide are variants of STAT6, particularly of human origin.

The GenBank accession numbers for human STAT proteins (STAT1 to STAT6) are as follows: STAT1 - NM_007315, STAT2 - NM_005419, STAT3 - NM_003150, STAT4 - NM_003151, STAT5A - NM_003152, STAT5B - NM_012448, and STAT6 - NM_003153. These identifiers correspond to the mRNA sequences of each protein.

The Protein database identifiers (UniProtKB/Swiss-Prot IDs) for human STAT proteins are as follows: STAT1 - P42224, STAT2 - Q9Y3U8, STAT3 - P40763, STAT4 - Q14765, STAT5A - P42229, STAT5B - P51692, STAT6 - P42226.

Also encompassed by the invention is a collection of at least two expression systems as specified in any one of the above aspects and embodiments.

Another aspect of the invention relates to a recombinant cell comprising an expression system according to any one of the above aspects and embodiments of the invention.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Description of the Figures

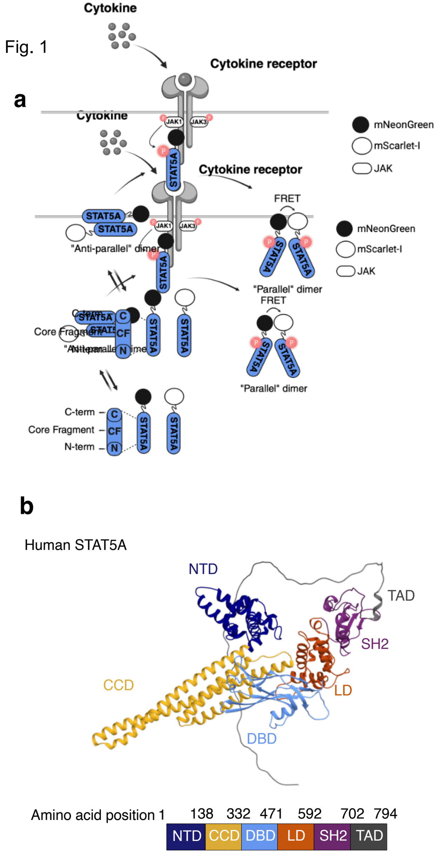

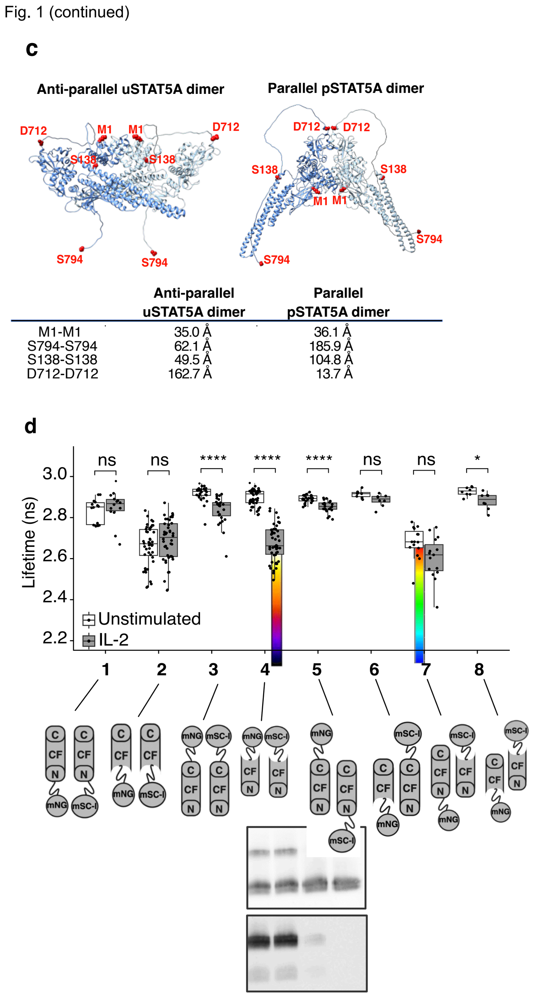

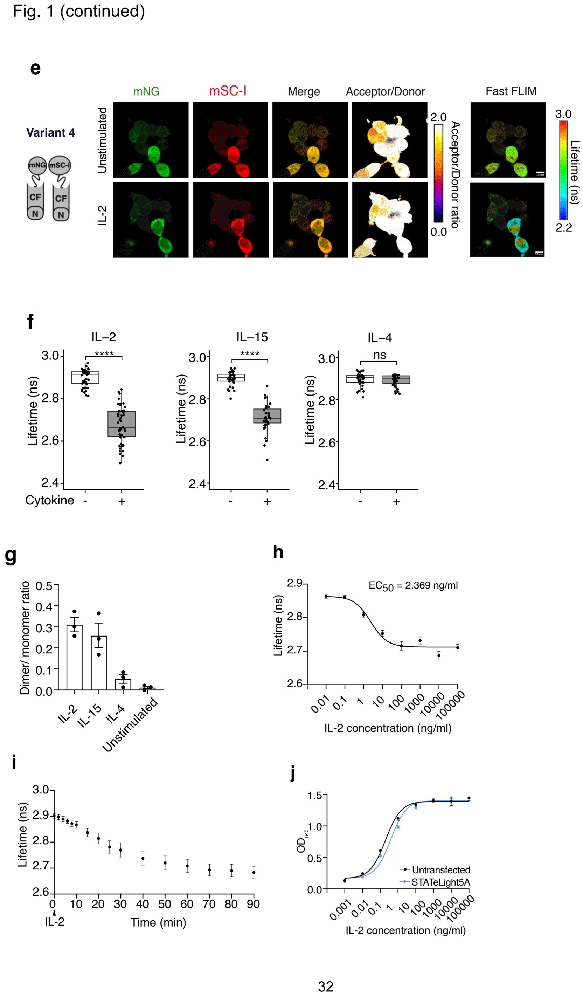

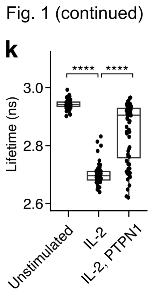

- Fig. 1: shows development and characterization of STAT5A biosensor using FLIM-FRET. (a) Schematic representation of FRET-based biosensor to detect STAT5A dimerization in the IL-2-JAK-STAT5 pathway. STAT5A monomers are fused to mNeonGreen (mNG) and mScarlet-I (mSC-I). Upon IL-2 stimulation, a switch in STAT5A mode of dimerization induces the change in the distance between mNG and mSC-I, resulting in FRET occurrence. (b) AlphaFold model of human STAT5A. N-terminal domain (NTD), dark blue, coiled-coil domain (CCD), yellow, DNA binding domain (DBD), light blue, linker domain (LD), orange, src-homology 2 domain (SH2), purple, C-terminal transactivation domain (TAD), grey. (c) AlphaFold models showing "parallel" and "anti-parallel" dimeric conformations of full-length STAT5A (ipTM = 0.642 and 0.611, respectively). Residues from which the distances were measured, red. (d) Quantification of mNG mean fluorescence lifetime of STAT5A screened variants transiently transfected in HEK-Blue^{™} IL-2 cells. Schematic cartoon represents STAT5A variants, NG= mNeonGreen, SC = mScarlet-I, N = N-terminal domain, CF = core fragment, C = C-terminal domain. Data were acquired on the same cells before (blue boxplots) and 30 min after (red boxplots) 3.5 µg IL-2 stimulation. From left to right, n = 14, 44, 28, 50, 24, 8, 15, 8 cells, respectively (Wilcoxon's rank-sum test, ****p<0.0001, ***p<0.001, **p<0.01, *0.05, n.s., not significant). (e) Representative images of the best-performing biosensor (STATeLight5A). (f) Quantification of mNG mean fluorescence lifetime on the same cells in response to IL-4 (n= 34), IL-15 (n=36), IL-2 (n=50). (g) Quantification of STAT5A dimers/monomers ratio by western blot using anti-STAT5A antibodies. (h) Dose-response curve of STATeLight5A. HEK-Blue^{™} IL-2 cells expressing STATeLight5A were stimulated with a titrated amount of IL-2 and mNG mean fluorescence lifetime was quantified. Shown are pooled mean fluorescence lifetime ± SEM from two independent experiments (n = 50-65 cells in each of IL-2 concentration). (i) mNG mean fluorescence lifetime of STATeLight5A expressed in HEK-Blue^{™} IL-2 cells was assessed over a time course of 90 min after IL-2 stimulation (n=12). Data are shown as mean ± SEM. Endogenous STAT5 signaling is not affected by STATeLight5A, as evaluated by HEK-Blue^{™} IL-2 reporter assay. (j) STATeLight5A-expressing HEK-Blue^{™} IL-2 cells were stimulated with 3.5 µg of IL-2, with or without transfection with PTPN1. mNG mean fluorescence lifetime was quantified. From left to right, n = 57, 62, 72 cells. **** corresponds to p < 0.0001, one-way ANOVA followed by Tukey's post-hoc test. Data are shown as mean± SEM.

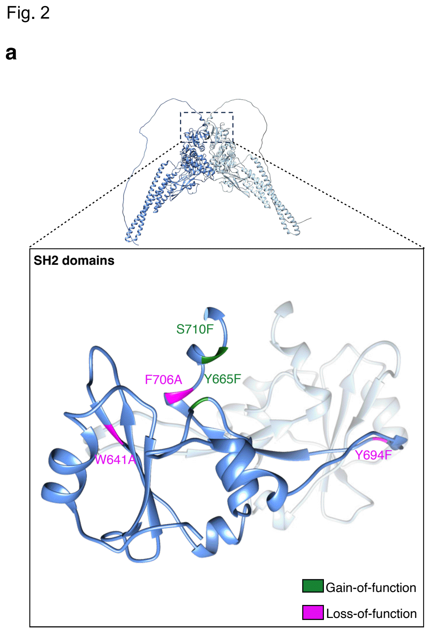

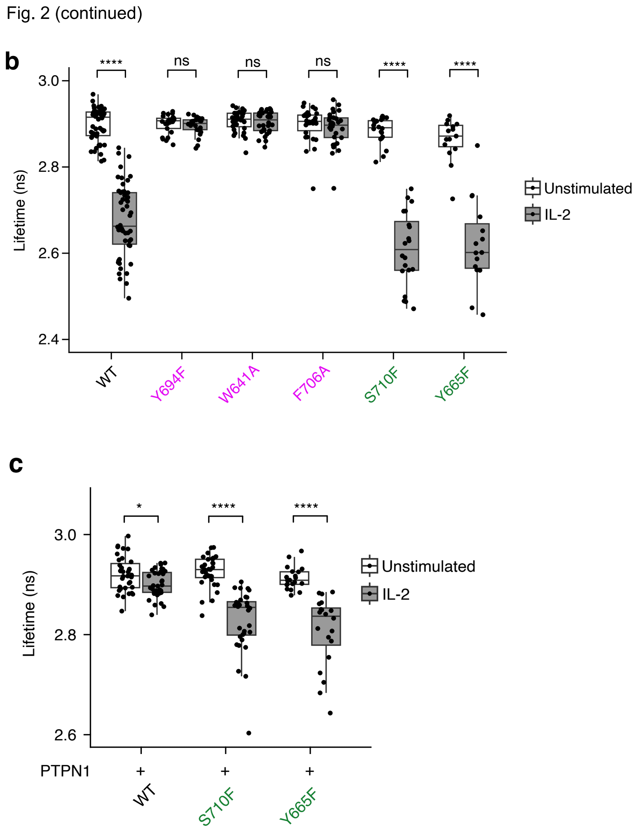

- Fig. 2: shows mutational analyses of STAT5A using the biosensor platform. (a) Schematics of two SH2 domains of STAT5A in the dimeric complexes. Loss-of-function (LOF) mutations and gain-of-function (GOF) mutations are indicated in green and red, respectively. Structure was predicted using AlphaFold simulation. (b) Quantification of mNG fluorescence lifetime in cells expressing LOF and GOF mutations. From left to right, n = 50, 33, 32, 30, 20, 15, 35 cells (Wilcoxon's rank-sum test, ****p<0.0001, ***p<0.001, **p<0.01, *0.05, n.s., not significant). (c) Quantification of mNG fluorescence lifetime in cells co-expressing GOF mutations and protein tyrosine phosphatase non-receptor type 1 (PTPN1).

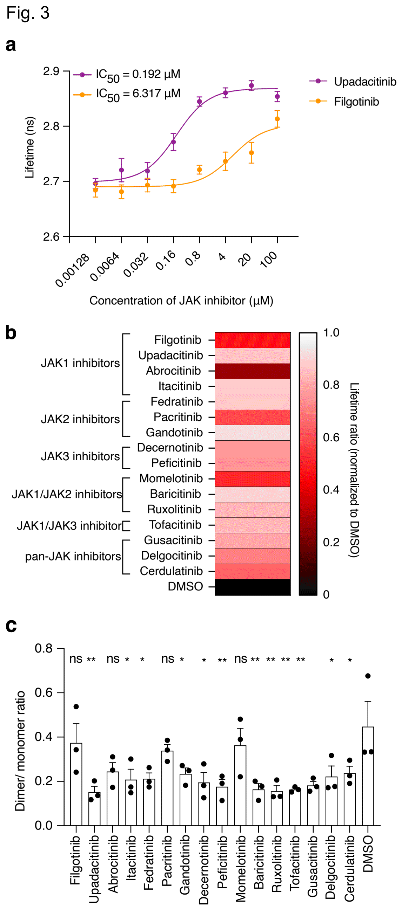

- Fig. 3: shows inhibitors screening using STATeLight5A. HEK-Blue^{™} IL-2 cells expressing STATeLight5A were treated with JAK inhibitors overnight, followed by stimulation with 3.5 µg of IL-2. (a) Dose-response curve of STATeLight5A treated with a titrated amount of Pacritinib (black) and Tofacitinib (blue). (n=15-30 cells in each inhibitor concentration). (b) Heatmap representation of the normalized lifetime ratio of all JAK inhibitors, compared to the untreated condition. mNG fluorescence lifetime ratio was calculated as $\frac{mNG Lifetime after IL - 2 stimulation}{mNG Lifetime before IL - 2 stimulation}$. The lifetime ratio of untreated cells was normalized to 0. (c) Quantification of STAT5A dimers/monomers in JAK inhibitor-treated cells ratio by western blot using anti-STAT5A antibodies. Differences between DMSO-treated condition and conditions treated with JAK inhibitors were analyzed using one-way ANOVA, followed by Dunnett's post-hoc test. *****p* < 0.0001, ****p* < 0.001, ***p* < 0.01, * *p* < 0.05, n.s., not significant.

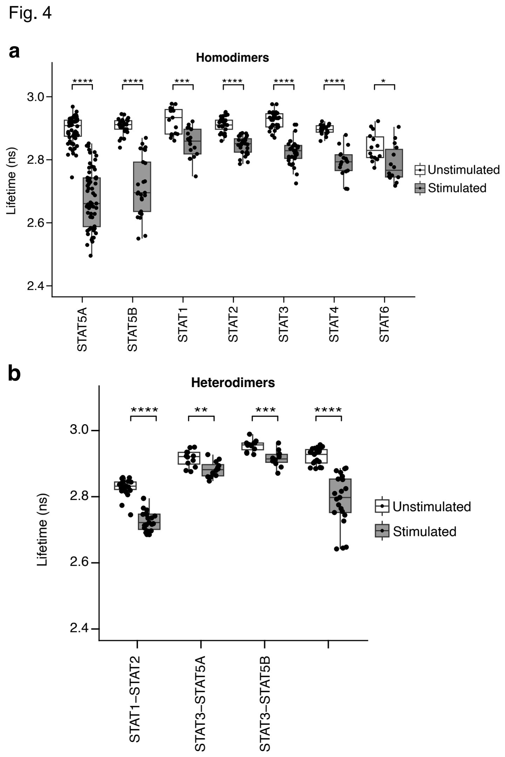

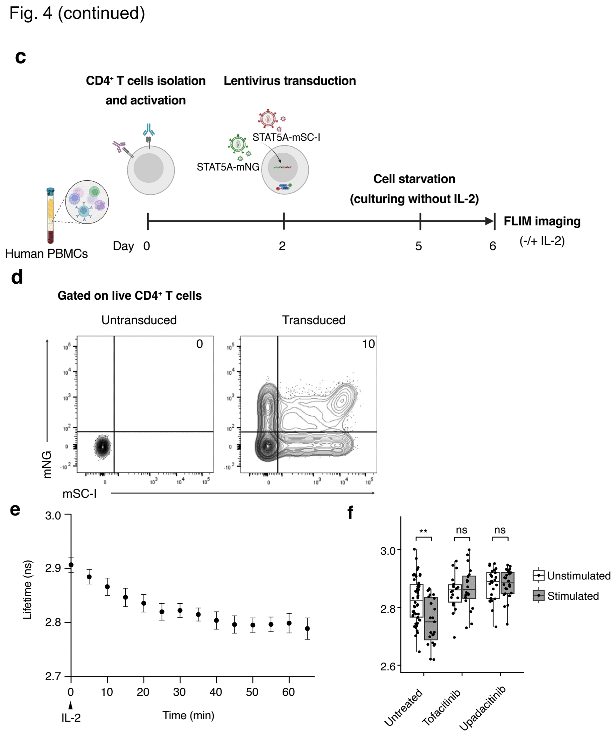

- Fig. 4: shows characterization of different homo- and heterodimeric STATeLight biosensors in cell lines and human primary CD4⁺ T cells. HEK-Blue^{™} IL-2 or HeLa cells were transfected with indicated homo- and heterodimeric STAT variants, including STAT1, STAT2, STAT3, STAT4, STAT5A, STAT5B and STAT6. mNG fluorescence lifetime was evaluated before and 30 min after stimulation with 3.5 µg of IL-2 (for STAT3, STAT5A and STAT5B), IFN-α (for STAT1, STAT2 and STAT4), and IL-4 (for STAT6). (a) Quantification of mNG mean fluorescence lifetime before and after cytokine stimulation of indicated homodimeric STATeLights. From left to right n= 64, 25, 14, 13, 20, 17, and 14 cells. (b) Quantification of mNG mean fluorescence lifetime before and after cytokine stimulation of indicated heterodimeric STATeLights. From left to right n= 23, 12, 12, and 21 cells. Wilcoxon's rank-sum test. (c) Schematic showing transduction of human primary CD4⁺ T cells with STATeLight5A, followed by stimulation with IL-2 and FLIM imaging. (d) Representative flow cytometry plots showing lentiviral transduction efficiency in human primary CD4⁺ T cells, with the mNG⁺mSC-I⁺ population corresponding to STATeLight5A-expressing cells. (e) mNG mean fluorescence lifetime of STATeLight5A in CD4⁺ T cells was assessed over a time course of 70 min after IL-2 stimulation (n= 10 cells). Data are shown as mean ± SEM. (f) Lentivirus-transduced CD4⁺ T cells were pretreated with Tofacitinib and Upadacitinib for 60 min and imaged before and after 30 min of IL-2 stimulation. Shown are pooled mean fluorescence lifetime ± SEM values of two independent experiments. ****p<0.0001, ***p<0.001, **p<0.01, *0.05, n.s., not significant.

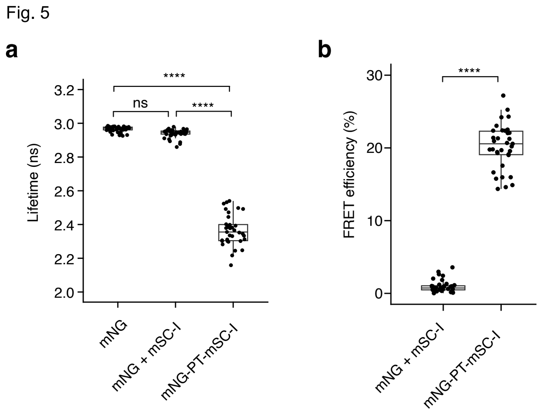

- Fig. 5: shows FLIM-FRET imaging of control constructs. (a) Quantification of mNG fluorescence lifetime and (b) FRET efficiency of HEK-Blue^{™} IL-2 cells transfected with mNG (n = 40), mNG and SC-I (n = 39), and mNG-mSC-I fusion protein (n = 35). One-way ANOVA followed by Tukey's post-hoc test. Data are shown as meant SEM. ****p < 0.0001.

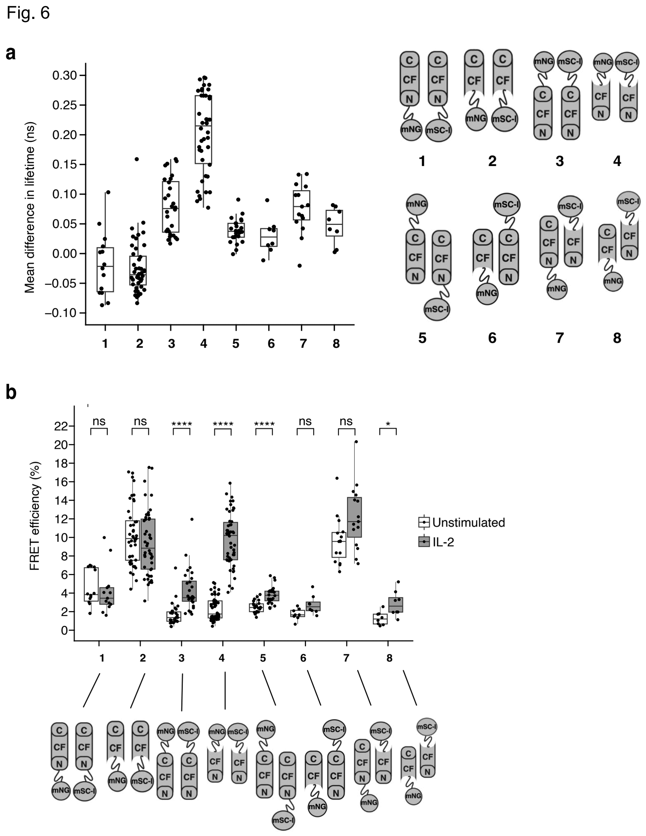

- Fig. 6: shows screened variants for FRET-based STAT5A biosensor. HEK-Blue^{™} IL-2 cells were transfected with different biosensor variants and mNG fluorescence lifetime was evaluated before and 30 min after stimulation with 3.5 µg of IL-2. (a) Mean difference in mNG fluorescence lifetime in the same cells before and after IL-2 stimulation. (b) FRET efficiency of different biosensor variants before and after IL-2 stimulation. Wilcoxon's rank-sum test, ****p<0.0001, ***p<0.001, **p<0.01, *0.05, n.s., not significant.

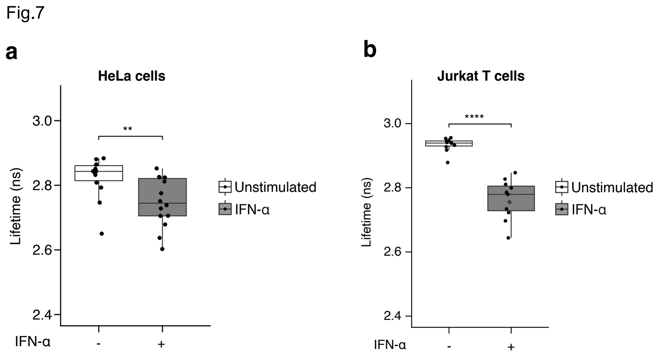

- Fig. 7: shows detection of STAT5A activation using STATeLight5A in HeLa and Jurkat T cells. Cells were transfected or transduced with STATeLight5A and mNG fluorescence lifetime was measured before and 30 min after stimulation with IFN-α. (**a**) Quantification of mNG mean fluorescence lifetime in HeLa cells (n = 14 cells). (**b**) Quantification of mNG mean fluorescence lifetime in Jurkat T cells (n = 11 cells). Wilcoxon's rank-sum test, **p<0.01, ****p<0.0001.

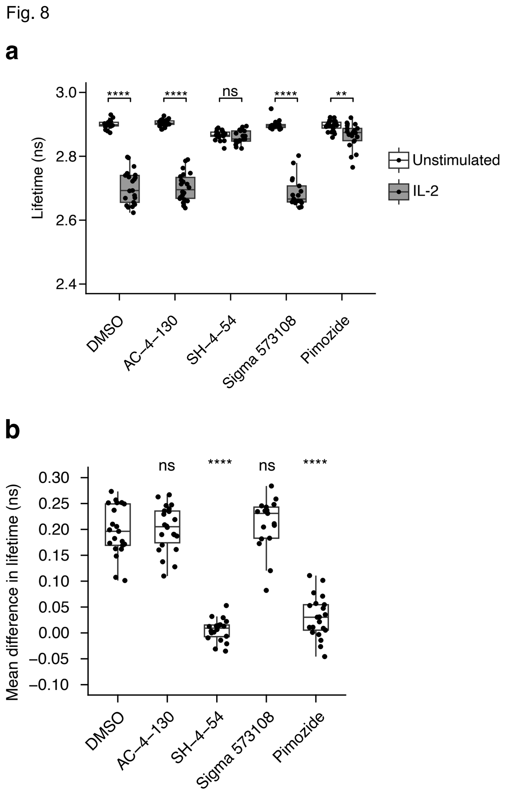

- Fig. 8: shows effect of STAT5 inhibitors on STAT5 dimerization. HEK-Blue^{™} IL-2 cells were transfected with STATeLight5A and incubated with indicated STAT5 inhibitors for 1 hour. Subsequently, cells were stimulated with 3.5 µg of IL-2 for 30 min and mNG fluorescence lifetime was measured. (**a)** Quantification of mNG fluorescence lifetime in cells treated with different STAT5A inhibitors. (**b**) Mean difference in mNG mean fluorescence lifetime. Differences between untreated and conditions treated with STAT5 inhibitors were analyzed using one-way ANOVA, followed by Dunnett's post-hoc test. *****p* < 0.0001, ****p* < 0.001, ***p* < 0.01, * *p* < 0.05, n.s., not significant.

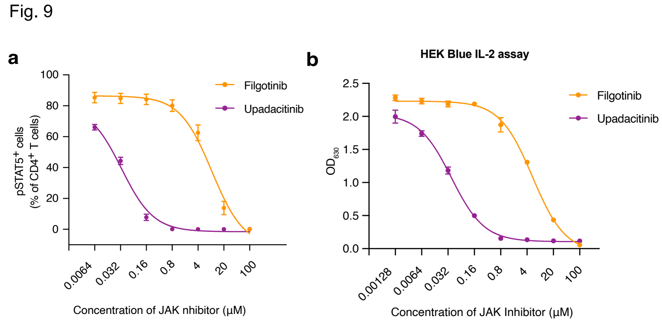

- Fig. 9: shows dose-response effects of Filgotinib and Upadacitinib on STAT5 dimerization. Human PBMCs or HEK-Blue^{™} IL-2 cells were pre-treated with titrated amounts of Filgotinib or Upadacitinib, followed by stimulation with IL-2. (**a**) Percentage of pSTAT5⁺ cells in CD4⁺ T cells treated with Filgotinib or Upadacitinib. Data are represented as mean ± SEM of n = 3 replicates. (**b**) Dose-response of HEK-Blue^{™} IL-2 cells treated with Filgotinib or Upadacitinib using HEK-Blue^{™} IL-2 reporter assay. Data are represented as mean ± SEM of n = 3 replicates.

### Examples

### Example 1: Biosensor engineering

We focused on engineering STAT5-based biosensor first, given the pivotal role of STAT5 in immune cell signaling. We hypothesized that tagging STAT5 monomers with a pair of fluorescent proteins (FP) at suitable positions would allow detection of cytokine-mediated conformational change from anti-parallel to parallel dimers and subsequent STAT5 activation (Fig. 1a). We selected mNeonGreen (mNG) and mScarlet-I (mSC-I) as the FRET pair of donor and acceptor fluorophores, respectively (Shaner, N. C. et al., Nat Methods, 10, (2013); Bindels, D. S. et al., Nat Methods, 14, (2017)). These fluorophores have favorable properties for detection by FLIM-FRET in live cells (McCullock, T. W., et al., PLoS One, 15, (2020)).

Upon signal activation, the positioning of mNG and mSC-I should induce a change in distance and orientation between donor and acceptor fluorophores, such that a detectable change in FRET efficiency can be observed.

The rational design of STAT5 biosensor is challenging because it must reliably differentiate between the two aforementioned dimeric conformations, namely anti-parallel and parallel. Furthermore, all available structures of mouse and human STAT5 consist of only the CF in the anti-parallel dimeric conformation. We tackled this problem by modeling the structure of a full-length STAT5A dimer using AlphaFold-multimer simulation (Fig. 1b, c). AlphaFold predicted STAT5A in anti-parallel and parallel dimeric conformations, which likely corresponded to its inactive and active states, respectively. To find the optimal fusion sites for mNG and mSC-I, we measured the distances between N- or C- termini of full-length STAT5A monomers. The distances between two C-termini (Ser794-Ser794) increased upon STAT5A conformational change, whereas they remained similar between the N-termini (Fig. 1c). However, the flexible region between the NTD and the CF, as well as the TAD of STAT5A were predicted with a low per-residue confidence score. For this reason, the assessment of the orientation and the distance of these domains remained inaccurate. To circumvent this inaccuracy, we fused mNG and mSC-I to truncated versions of STAT5A containing the CF plus either the N- or C-terminus of STAT5A and compared them to fusion proteins containing full-length STAT5A. The distance between two CCDs (Ser138-Ser138) were approximately 50 Å and 105 Å in anti-parallel and parallel dimers, respectively. Thus, we expected to detect higher FRET efficiency in the anti-parallel dimer conformation. As evident from the measured distances in STAT5A models and other available STAT crystal structures, parallel dimerization is expected to bring SH2 domains in proximity to each other. Hence, we postulated that direct fusion of FPs C-terminally to SH2 domains (Asp712-Asp712) would lead to efficient FRET upon STAT5A activation.

To experimentally validate our hypothesis, we first tested the compatibility of mNG and mSC-I in our FLIM-FRET settings. To this end, we generated an mNG-mSC-I fusion protein connected via a rigid, two-amino-acid proline-threonine (Pro-Thr) linker. The rigidity of the linker is not strictly important, we tried a GS linker and it also worked. This fusion protein exhibited on average 20.4% FRET efficiency, thus demonstrating a high suitability of the mNG-mSC-I FP pair for studying protein-protein interactions (Fig. 5). Next, we generated full-length and truncated STAT5A variants that were tagged with mNG or mSC-I at their N- or C-termini. Subsequently, we performed a comprehensive screening by co-transfecting eight different combinations of mNG and mSC-I-tagged STAT5A variants into HEK-Blue^{™} IL-2 cells. This interleukin 2 (IL-2)-sensitive cell line harbors a functional IL-2 receptor (IL-2R)-JAK1/3-STAT5 signaling pathway, thus allowing the evaluation of STAT5 activation. Upon IL-2 treatment, we expected a change in mNG fluorescence lifetime as our FRET read-out. Fusion of mNG and mSC-I to full-length STAT5A at the N-terminus (variant 1) did not lead to any significant change in fluorescence lifetime (Fig. 1d, e, and Fig. 6). Notably, when FPs were directly linked N-terminally to the CCD of STAT5A (variant **2**), FRET was already observed at the unstimulated state, indicative of formation of inactive anti-parallel dimers. C-terminal fusion of mNG and mSC-I to both full-length and truncated STAT5A (variants **3** and **4**, respectively) resulted in a significant decrease in fluorescence lifetime following IL-2 stimulation. FRET efficiency in variant **4** was particularly evident, resulting in up to 12%, which is consistent with the predicted close distance between SH2 domains in the parallel conformation. Variants **5-8**, in which one STAT5A was labeled at the N-terminus and the other at the C-terminus, led to a modest change in mNG fluorescence lifetime upon IL-2 stimulation. Since variant **4** exhibited the highest FRET efficiency upon IL-2 stimulation, we selected it as our best-performing biosensor to detect STAT5A activation and named it STATeLight5A. To further demonstrate the utility of STATeLight5A in measuring STAT5A activation by other cytokine-mediated signaling pathways, we treated HeLa and Jurkat T cells expressing our biosensor with IFN-α. Indeed, both cells expressing STATeLight5A showed an increased FRET efficiency upon IFN-α stimulation (Fig. 7).

### Example 2: STATeLight5A characterization

To characterize the molecular specificity and dynamic response of STATeLight5A, we stimulated STATeLight5A-expressing cells with IL-2, IL-15, and IL-4. IL-15 activates STAT5 through a dimeric receptor consisting of IL-2Rβ and the common cytokine receptor γ chain. Thus, stimulation of cells with IL-15 significantly decreased mNG fluorescence lifetime, comparable to IL-2 stimulation. Conversely, mNG fluorescence lifetime was not affected by IL-4 stimulation (Fig. 1f), since IL-4 does not signal through the IL-2 receptor.

To confirm STAT5A dimerization and phosphorylation in response to these cytokines, we performed native polyacrylamide gel electrophoresis (PAGE) coupled with western blot (WB) using antibodies specific for STAT5A and pSTAT5. Unlike stimulation by IL-4, both IL-2 and IL-15 potently induced STAT5A dimerization (Fig. 1g). Moreover, using STATeLight5A, we examined the dose response of IL-2 on STAT5A activation and obtained an EC₅₀ value of 2.369 ng/ml. We detected IL-2-mediated STAT5A dimerization at a concentration as low as 0.01 ng/ml, while saturation of signal was observed at around 100 ng/ml (Fig. 1h). To study the dynamics of STAT5A activation, we treated STATeLight5A-expressing HEK-Blue^{™} IL-2 cells with a saturating dose of IL-2 and tracked the lifetime of mNG over a time course of 90 minutes. Formation of dimeric STAT5A was detected within 5 minutes of IL-2 stimulation and reached a maximum after 70 minutes (Fig. 1i). To test whether STATeLight5A affected endogenous STAT5 signaling, we measured activation of this pathway by the HEK-Blue^{™} IL-2 reporter assay. Compared to untransfected cells, endogenous STAT5 signaling did not change markedly in cells expressing STATeLight5A, indicating no major interference of our biosensor (Fig. 1j). To test whether our biosensors can be used to evaluate molecular events in a reversible manner, we stimulated STATeLight5A-expressing HEK-Blue^{™} IL-2 cells with IL-2 followed by transfection with protein tyrosine phosphatase 1 (PTPN1). We observed a reduction in mNG fluorescence lifetime upon IL-2 stimulation, which was reversed by transfection of PTPN1, indicating the utility of STATeLight5A to also measure STAT5 dephosphorylation (Fig. 1k). Altogether, STATeLight5A is a highly sensitive biosensor to monitor STAT5 activation in live cells.

### Example 3: Mutational analyses of STAT5 using biosensor platform

STAT5A parallel dimerization is driven by reciprocal interactions between pTyr694 and different amino acids located in the SH2 domain. Typically, mutations at these sites either abrogate or further stabilize STAT5 dimers and can thus be frequently observed in various pathological conditions, including human leukemia. To study how STAT5A dimerization could be modulated, we selected and generated three loss-of-function (LOF) mutations, namely Y694F, F706A and W641A, as well as two gain-of-function (GOF) mutations, namely S710F and Y665F (Fig. 2a). Subsequently, we checked for changes in mNG fluorescence lifetime upon IL-2 stimulation of STATeLight5A-expressing cells that harbored these single-point mutations. Compared to wild-type (WT) STATeLight5A, IL-2-treated LOF mutants showed no significant change in mNG fluorescence lifetime (Fig. 2b). Meanwhile, the tested GOF mutants showed even slightly higher FRET efficiency compared to WT STATeLight5A (Fig. 2b). Similarly, using native PAGE-WB analysis, we observed LOF mutants were found only in the monomeric form. In contrast, STAT5A GOF mutants displayed in both dimeric and monomeric forms, thus confirming the observation by FLIM-FRET measurement. Notably, GOF mutants are more resistant to dephosphorylation compared to WT STAT5. Thus, we hypothesized that co-transfecting PTPN1 with these GOF mutants would result in higher STAT5 activation upon IL-2 stimulation compared to WT STATeLight5A. Indeed, these GOF mutants showed a higher increase in FRET efficiency, confirmed by native PAGE-WB (Fig. 2c). Collectively, we demonstrated different STAT5 dimerization efficiencies in different disease-relevant STAT5 mutants.

In addition to Tyr phosphorylation at residue 694, STAT5 activity is also regulated by several post-translational modifications such as serine/threonine phosphorylation, acetylation, SUMOylation, ubiquitination, and O-GIcNAcylation. Nevertheless, the influence of such modifications on STAT5 dimerization, particularly the phosphorylation of tyrosine residues other than Tyr 694, remains elusive. Thus, we set out to investigate whether these Tyr residues could potentially affect IL-2-mediated STAT5 dimerization. To this end, we systematically introduced Tyr to Phe mutations in STATeLight5A and examined their capability to dimerize by FLIM-FRET. Compared to WT STATeLight5A, Y22F and Y683F showed less reduction in mNG fluorescence lifetime upon IL-2 stimulation. Conversely, the Y171F mutation exhibited a higher decrease in mNG fluorescence lifetime compared to WT. Based on these observations, we speculate that Tyr22, Tyr683, and Tyr171 play a role in STAT5 activation, either as phosphorylation carriers or allosteric modulators. Overall, these mutational studies showcased the utility of STATeLight5A to facilitate various STAT5 structural and functional studies, which enable the investigation of different disease conditions related to STAT5 activation.

### Example 4: Validation of STATeLight5A as an inhibitor screening platform

Constitutive STAT5 activation resulting from excessive JAK activation or STAT5 gain-of-function mutation is commonly observed in different types of cancer. Thus, significant efforts have been dedicated to developing new drugs, interfering with the overactivated JAK-STAT5 pathway. Although JAK inhibitors are extensively used to treat inflammatory diseases and cancer, developing new JAK inhibitors with minimal adverse side effect profiles remains an unmet clinical need. Novel inhibitors directly targeting STAT5 dimerization have recently emerged as an alternative promising therapeutic strategy, yet none of these preclinical STAT5 inhibitors have been advanced to clinical trials. We used STATeLight5A to test the efficacy of available STAT5 inhibitors in blocking STAT5A dimerization. Among all tested STAT5 inhibitors, SH-4-54 completely disrupted STAT5A dimerization, as evidenced by little to no change in mNG fluorescence lifetime and native-PAGE-WB analysis. However, other compounds exhibited only a limited level of inhibition of STAT5A dimerization (Fig. 8).

Since library screening is a common approach in drug discovery, we sought to establish STATeLight5A as a medium-to-high-throughput screening platform for potential JAK and STAT5 inhibitors. To set up the optimal condition for our screening, we first performed a dose-response study with JAK inhibitors Filgotinib and Upadacitinib. Both drugs showed moderate to high inhibition of IL-2-induced STAT5A dimerization when used at doses of 0.16-4 µM (Fig. 3a). In addition, we confirmed kinetic characteristics of these inhibitors via HEK-Blue^{™} IL-2 reporter assay and intracellular staining of pSTAT5 in human CD4⁺ T cells (Fig. 9). Consequently, we set up a proof-of-concept drug screening, in which cells expressing STATeLight5A were treated with a collection of JAK inhibitors. Cells exposed to JAK inhibitors showed moderate to little decrease in mNG fluorescence lifetime upon IL-2 stimulation, compared to untreated cells, indicating moderate to high suppression of STAT5A dimerization (Fig. 3b). These findings were confirmed by using native PAGE-WB analysis (Fig. 3c). Taken together, our biosensor proved to be a useful tool to screen for new inhibitors targeting the JAK-STAT signaling pathway and to quantitate the potency of existing drugs.

### Example 5: Application of biosensor design strategy to other STATs

Given the homology between STAT5A and other STATs, we wondered whether a similar biosensor design could be applied to other STATs. Based on protein sequence, structural alignment and available STAT structures, we engineered other STAT biosensors and evaluated their utility in detecting STAT homo- and heterodimerization. We detected efficient FRET responses in HEK-Blue^{™} IL-2 and HeLa cells expressing homodimeric STATeLight5B and STATeLight1-4 biosensors upon stimulation with IL-2 and IFN-α, respectively (Fig. 4a). Additionally, STATeLight6 biosensor showed a FRET signal upon IL-4 stimulation. As for STAT heterodimers, we observed moderate signals with STAT1/2, STAT3/5A and STAT3/5B, while STAT5A/5B exhibited the highest FRET efficiency (Fig. 4b). Overall, these results demonstrated the suitability and applicability of our approach to other STAT members.

### Example 6: STAT5A sensing in primary CD4⁺ T cells

Finally, to examine STAT5 activation in primary immune cells, we transduced human CD4⁺ T cells with STATeLight5A using lentiviral particles (Fig. 4c). Upon IL-2 stimulation, we observed STAT5 activation, demonstrating the use of our biosensor to track real-time T cells activation (Fig. 4d, e). Furthermore, using STATeLight5A-transduced CD4⁺ T cells, we confirmed inhibition of the JAK-STAT5 pathway by the JAK inhibitors Tofacitinib and Upadacitinib (Fig. 4f). In summary, our biosensor design opens up new possibilities to evaluate STAT5 activation in primary immune cells.

### Example 7: Discussion

Measuring STAT activation in real time is of great use to study the dynamics of JAK-STAT signaling in its native context. Additionally, such biosensors can serve as a screening platform for drug discovery. Here we report on the molecular engineering and characterization of a novel STAT5A biosensor, termed STATeLight5A. FLIM-FRET-based STATeLight5A enabled the continuous and real-time visualization of STAT5 activation by directly detecting its conformational change in live cells. The herein discovered principles of molecular design of the STATeLight5A biosensor also extend to other homo- and heteromeric STATs, including STAT1-4, STAT5B and STAT6. In addition, STATeLight5A is a useful tool to study the effect of disease-related mutants of the IL-2-JAK1/3-STAT5 signalling pathway. These may include not only STAT5 mutations but also mutations occurring in various cell surface receptors, kinases, phosphatases and other post-translational modifiers. Importantly, our biosensor platform allows for the screening of new drug candidates, as demonstrated by our proof-of-concept JAK inhibitor screening. Finally, the knowledge gained from our developments can be used to generate new tools for simultaneous visualization of multiple related events, thus enabling us to achieve a better understanding of the information flow in the complex signaling network. Altogether, our findings will not only enhance the understanding of the underlying mechanism of JAK-STAT activation, but also facilitate the development of better diagnostic tools and drug screening strategies.

### Example 8: Methods

### Construct design

Plasmids encoded for mNeonGreen (pmNeonGreenHO-G, #127912) and mScarlet-I (pLV-mScarlet-I, #159172) were obtained from Addgene (gifted by Isei Tanida and Pantelis Tsoulfas, respectively. Plasmid encoded for human PTPN1 was obtained from Sino Biological (HG10304-UT). Full length STAT5A fused to mNeonGreen and mScarlet-I at the C- or N-terminus and mNG-PT-mSC-I constructs were synthesized by Twist Bioscience. Of these STAT5A constructs, truncated variants were generated using Q5 Site-Directed Mutagenesis Kit (NEB). KLD-treated PCR product was subsequently transformed into NEB 5-alpha competent *E.coli* cells (NEB). Bacterial colonies were isolated and sequenced using Sanger sequencing (Ecoli NightSeq, Microsynth AG). Plasmids were extracted using GenElute^{™} Plasmid Miniprep Kit (Sigma-Aldrich) or NucleoBond^{®}Xtra Maxi Plus (Machery-Nagel) according to the supplier protocol. Plasmid DNA concentration was measured using NanoDrop spectrophotometer (Thermo Scientific). Constructs containing gain-of-function, loss-of-function and Tyr-to-Phe mutations were generated using a similar site-directed mutagenesis procedure. Primers were synthesized by Microsynth.

### Cell culture and primary cells

Human embryonic kidney (HEK)-Blue^{™} IL-2 cells obtained from InvivoGen were cultured in Dulbecco's Modified Eagle Medium (DMEM) supplemented with 10% fetal bovine serum (FBS; 10%, v/v, Gibco), penicillin-streptomycin (100U/ml, Gibco), L-glutamine (2mM, Gibco), Normocin^{™} (100 µg/ml, InvivoGen), HEK-Blue^{™} CLR Selection (1X, InvivoGen) and puromycin (1µg/ml, InvivoGen). The HeLa cell line was a kind gift from Prof. Thorsten Zenz (University Hospital Zurich, Zurich, Switzerland). HeLa cells were cultured in advanced DMEM supplemented with 10% FBS, penicillin-streptomycin and L-glutamine. Jurkat cells obtained from ATCC were cultured in RPMI medium (Gibco) supplemented with 10% FBS, penicillin-streptomycin (100U/ml) and L-glutamine (2mM).

Human peripheral blood was collected from healthy individuals and peripheral blood mononuclear cells (PBMCs) were isolated by Ficoll-Paque PLUS (GE Healthcare) gradient centrifugation. Prior informed consents were collected and approved by the Ethical Committee of Canton of Zurich (BASEC no. 2016-01440).

### Cell transfection

Effectene Transfection Reagent Kit (Qiagen) was used for cell transfection. On day 0, HEK Blue^{™} IL-2 cells were seeded in either µ-Dish 35mm, low, µ-Plate 24 well plates (ibidi), or TPP^{®} 6-well plates. On day 1, plasmid DNA was prepared in Buffer EC, followed by the addition of Enhancer (1:8 DNA to Enhancer ratio). This mixture was incubated for 5 min, then Effectene was subsequently added (1:8 DNA to Effectene ratio). The mixture was further incubated at room temperature for 10 min, then mixed with cultured medium with a 1:1 ratio and added dropwise to cells. On day 2, cells were replaced with fresh DMEM. Cells were incubated at 37°C in a 5% CO₂ humidified incubator for 2 days.

### Lentivirus transduction

Plasmids pLV-STAT5-mNG and pLV-STAT5-mSC-I were synthesized and VSV-G pseudotyped third-generation lentiviral particles were produced by VectorBuilder. Human CD4⁺ T cells were isolated (MojoSort^{™} Human CD4 T Cell Isolation Kit) and activated for 48 h with anti-CD3 (clone OKT3), anti-CD28 (clone CD28.2) in RPMI medium supplemented with IL-2 (10 ng /ml). Cells were then infected with concentrated lentiviral particles at a MOI of 50 on RetroNectin^{®}-coated plates. Fresh RPMI medium supplemented with IL-2 was replaced after 60 h. Before the imaging, cells were starved overnight in RPMI medium without IL-2.

### Live cells imaging

Experiments were conducted with transfected HEK-Blue^{™} IL-2 cells in µ-Dish 35mm, low or µ-Plate 24 well (ibidi). Cells were maintained in FluoroBrite^{™} DMEM (Gibco) in a humidified incubator at 37°C, 5% CO₂ during the imaging session. FLIM imaging was performed with Confocal Laser Scanning Microscope - Leica SP8 inverse FALCON using an 63X 1.3NA glycerin immersion objective. To record fluorescent intensity, cells were excited sequentially with white light laser (WLL) at 488 nm and 569 nm (laser power from 2 to 5%) and detected at 499-558 nm and 577-605 nm, respectively with HyD detectors using the photon counting mode. The fluorescent lifetime of mNeonGreen was collected using a time correlated single photon counting module. Photons were collected by pulsed WLL at 40 MHz using Filter Supported WLL excitation line (488nm) and 499-558 nm emission. For FLIM imaging setting, zoom 2.0, pixel format of 512x512, 16-line repetitions were used. Cells were imaged before and 30 min after IL-2 stimulation.

### Native polyacrylamide gel electrophoresis (PAGE)

Transfected HEK-Blue^{™} IL-2 cells were lysed on ice using NativePAGE^{™} Sample Buffer, supplemented with Digitonin (1%, all from NativePAGE^{™} Sample Prep Kit, Thermo Fischer Scientific). Cell lysates were centrifuged at 17,000 g for 30 min at 4°C and the protein concentration was measured by Bicinchoninic Acid (BCA) protein assay (Pierce^{™} BCA Protein Assay Kit, Thermo Scientific^{™}). 30 µg of cell lysate was incubated with NativePAGE 5% G-250 Sample Additive and separated on NativePAGE^{™} 4 to 16%, Bis-Tris, 1.0 mm, Mini Protein Gel, 10-well (all from Thermo Fischer Scientific) at 150V in room temperature.

### Western blot

Native gel was transferred to a 0.2µm polyvinylidene fluoride (PVDF) membrane (Trans-Blot^{®} Turbo^{™} Mini PVDF Transfer Packs, Bio-rad) using Trans-Blot^{®} Turbo^{™} Transfer System (Bio-rad). The membrane was blocked with 5% skim milk in 0.1% Tween 20 in Tris buffer saline pH 7.4 (TBS-T) for 2 h, followed by incubation with primary antibodies overnight and subsequently secondary anti-Horseradish Peroxidase (HRP) antibodies incubation for 2 h. Primary antibodies including phospho-STAT5 (Tyr694) antibody (Cell Signaling, #9351, 1:3000 dilution), STAT5A Monoclonal antibody (TrueMAB^{™}, ThermoFisher, clone OTI4E7, 1:3000 dilution), anti-Actin antibody (Merck, #MAB1501R, clone C4, 1:1000 dilution) and secondary antibodies including anti-rabbit IgG, HRP-linked Antibody (Cell Signaling, #7074, 1:4000 dilution) and goat anti-mouse HRP-conjugated Antibody (Bethyl, #A90-116P, 1:2000 dilution) were diluted in 5% skim milk in TBS-T prior to use. This protocol was done using BlotCycler^{™} Mini (Precision Biosystems). The membrane was developed using Pierce ECL Western Blotting Substrate (Thermo Fischer) and imaged by chemiluminescence using ChemiDoc^{™} Imaging System (Bio-rad).

### Inhibitors screening

HEK-Blue^{™} IL-2 cells were transfected with STATeLight5A in µ-Plate 24 well plate. For STAT5 inhibitors study, cells were incubated with 10 µM of AC-4-130 (MedChemExpress), SH-4-54 (Cayman Chemical), pimozide (Sigma Aldrich), 573108 (Sigma-Aldrich) for 1 h. For JAK inhibitors studies in HEK-Blue IL-2 and primary CD4⁺ T cells, cells were incubated with 0.8 µM of JAK inhibitors (MedChemExpress) overnight or 1 h, respectively.

### Image analysis and quantification

The ratio of mScarlet-I to mNeonGreen fluorescent intensity was quantified using a custom-written macro linked to Fiji. Cells with a ratio of mSC-I/mNG fluorescence larger than 1.5 were picked for fluorescent lifetime determination. The cytoplasm of each cell was selected as a region of interest (ROI) and subjected to n-exponential reconvolution lifetime fit by LasX FLIM software (Leica).

### HEK-Blue^{™} IL-2 reporter assay

5x10⁴ HEK-Blue^{™} IL-2 cells per well were seeded in a 96-well flat-bottom plate. Cells were incubated with a titrated concentration of Filgotinib or Upadacitinib for 1 h. Subsequently, 0.31 ng/ml IL-2 was added to each well and the plate was incubated for 20 h at 37°C, 5% CO₂. 20µl of cell supernatant per well was incubated with 180 µl of QUANTI-Blue^{™} Solution (InvivoGen). STAT-inducible secreted embryonic alkaline phosphatase (SEAP) level was measured using Infinite^{®} 200 PRO (Tecan) at 630 nm.

### pSTAT5 measurement by flow cytometry

To measure pSTAT5 in human CD4+ T cells ex vivo, 5x10⁵ human peripheral blood mononuclear cells were seeded in 96-well U-bottom plates, and stained with the viability dye (Zombie UV450) with the addition of FcR blocking reagent for 20 min at 4°C. After washing with FACS buffer (phosphate-buffered saline supplemented with 2% fetal bovine serum and 0.4% ethylenedinitrilotetraacetic acid), cells were stained with anti-CD3 (BUV805), anti-CD4 (BUV496), anti-CD8 (BUV615), and anti-CD19 (BV605) in diluted Brilliant stain buffer (BD Biosciences, cat. 563794) at room temperature. Following the washing, cells were stimulated with 1000 ng/ml of IL-2 in the presence of titrated concentrations of JAK inhibitors for 20 min at 37°C. Subsequently, cells were incubated with warm Phosflow^{™} fix buffer I (BD Biosciences, cat. 557870) for 10 min at 37°C. Subsequently, cells were permeabilized with Phosflow^{™} perm buffer III (BD Biosciences, cat. 558050) for 1 h at 4°C. Finally, after washing, cells were stained with α-pSTAT5 (AlexaFluor 488) for 1 h at room temperature and acquired by Cytek Aurora spectral flow cytometer (Cytek Biosciences). Data were analyzed using FlowJo software.

### Statistics

All graphs were plotted, and statistical tests were performed using either GraphPad Prism version 9 or RStudio. Pair-wise comparisons of fluorescent lifetime before and after IL-2 stimulation were performed using Wilcoxon's rank-sum test with FDR-adjusted p-value. For multiple groups comparison, one-way ANOVA followed by Tukey's multiple comparisons test was performed. For all performed statistical tests, ****p<0.0001, ***p<0.001, **p<0.01, *0.05, n.s., not significant.

### Cited references:

Letra-Vilela, R. et al., FASEB Bioadv 2, 116-125 (2020).
Okada, Y. et al., Sci Rep 8, 2385 (2018).
Neculai, D. et al., Journal of Biological Chemistry 280, 40782-40787 (2005).
Shaner, N. C. et al., Nat Methods 10, 407-409 (2013).
Bindels, D. S. et al., Nat Methods 14, 53-56 (2017).
McCullock, T. W., et al., PLoS One 15, e0219886 (2020).

All scientific publications and patent documents cited in the present specification are incorporated by reference herein.

## Claims

1. A method for observing signal transducer and activator of transcription (STAT) dimerization in a cell ex-vivo, said method comprising the steps of:
a. providing a cell that expresses a first variant of a STAT polypeptide and a second variant of a STAT polypeptide, wherein the first variant is attached to a first peptide moiety, and the second variant is attached to a second peptide moiety,
wherein at least one of the first variant and the second variant consists of a truncated STAT polypeptide,
wherein the first peptide moiety and the second peptide moiety form a Förster resonance energy transfer (FRET) pair comprising a donor and an acceptor;
b. measuring Förster resonance energy transfer (FRET) between the first fluorescent moiety and the second fluorescent moiety.

2. A method for determining a modulation of STAT dimerization, said method comprising:
a. observing STAT dimerization in a cell according to claim 1, wherein the cell is exposed to a stimulus;
b. comparing the STAT dimerization to a control wherein the stimulus is absent; particularly wherein the stimulus is the presence of a compound.

3. The method according to any one of the preceding claims, wherein the first variant and the second variant are both truncated on their C terminus.

4. The method according to claim 6, wherein the first variant does not comprise the C-terminal domain, and the second variant does not comprise the C-terminal domain, particularly wherein the first variant is constituted of all N-terminal domains of the STAT protein except the C-terminal domain, and the second variant is constituted of all N-terminal domains of the STAT protein except the C-terminal domain.

5. The method according to any one of the preceding claims, wherein the first variant is attached to the first fluorescent moiety at the C terminus of the first, truncated STAT variant, and the second variant is attached to the second fluorescent moiety at the C terminus of the second, truncated STAT variant.

6. The method according to any one of the preceding claims, wherein the first variant and the second variant both consist of a C-terminally truncated STAT protein and are both ligated each to a fluorescent protein moiety.

7. The method according to claim 6, wherein the fluorescent protein moiety is linked to the C-terminally truncated STAT protein through a peptide linker 1 - 4 amino acids in length.

8. The method according to any one of the preceding claims, wherein the first variant and the second variant are expressed from different nucleic acid expression vectors.

9. The method according to any one of the preceding claims, wherein an expression level of the first variant being attached to a donor fluorophore is between 1% and 95% of an expression level of the second variant being attached to an acceptor fluorophore.

10. The method according to any one of the preceding claims, wherein the first variant and the second variant are selected from
a. variants of STAT5A;
b. variants of STAT5B;
c. variants of STAT1;
d. variants of STAT2;.
e. variants of STAT3;
f. variants of STAT4; and
g. variants of STAT6.

11. An expression system comprising:
a. a first expression cassette comprising a nucleic acid sequence encoding a first variant of a STAT polypeptide covalently linked to a first peptide moiety and
b. a second expression cassette comprising a nucleic acid sequence encoding a second variant of the same STAT polypeptide covalently linked to a second peptide moiety,
wherein at least one of the first variant and the second variant consist of a truncated STAT polypeptide, and
wherein the first fluorescent moiety and the second peptide moiety can form a Förster resonance energy transfer (FRET) pair comprising a donor and an acceptor moiety;
wherein both the first and second expression cassette are under control of a promoter sequence operable in a mammalian cell.

12. The expression system according to claim 11, wherein the expression cassette expressing the acceptor fluorophore expresses at higher expression rate than the expression cassette expressing the donor fluorophore.

13. The expression system according to any one of claims 11 to 12, wherein the first variant and the second variant both consist of a C-terminally truncated STAT protein and are both ligated each to a fluorescent protein moiety.

14. The expression system according to any one of claims 11 to 13, wherein the first variant and the second variant are selected from:
a. variants of STAT5A.
b. variants of STAT5B.
c. variants of STAT1.
d. variants of STAT2.
e. variants of STAT3.
f. variants of STAT4.
g. variants of STAT6.

15. A collection of at least two expression systems as specified in any one of claims 11 to 14.
